Europäisches Patentamt
European Patent Office
Office européen des brevets

(19) (11) Publication number: **0 007 759**
**A1**

(12)
# EUROPEAN PATENT APPLICATION

(21) Application number: 79301400.2

(22) Date of filing: 16.07.79

(51) Int. Cl.³: G 01 N 23/222
G 01 N 33/28

(30) Priority: 21.07.78 GB 3070278

(43) Date of publication of application:
06.02.80 Bulletin 80/3

(84) Designated Contracting States:
DE FR GB NL

(71) Applicant: United Kingdom Atomic Energy Authority
Patents Branch 11 Charles II Street
London SW1Y 4QP(GB)

(72) Inventor: Clayton, Colin Geoffrey
Sprole Cottage, Barrow Road
Shippon, Abingdon Oxfordshire(GB)

(72) Inventor: Merrill, Howard Nolan
12 Lowther Road
Millom, Cumbria(GB)

(74) Representative: Wood, Paul Austin
Patents Branch United Kingdom Atomic Energy
Authority 11 Charles II Street
London SW1Y 4QP(GB)

(54) **Method of and apparatus for measuring the water content of crude oil.**

(57) A method of measuring the water content of crude oil comprising the operations of irradiating a sample of crude oil in a fixed geometry with a known dose of neutrons measuring the amount of $\gamma$-ray activity arising from the interaction of the neutrons with either chlorine or oxygen present in the sample, deriving the specific activity of the $\gamma$-rays from the sample, and comparing the specific activity of the $\gamma$-rays from oxygen or chlorine in the sample with that arising from a known concentration of oxygen or chlorine in crude oil, and an apparatus for carrying out the method.

FIG. 1.

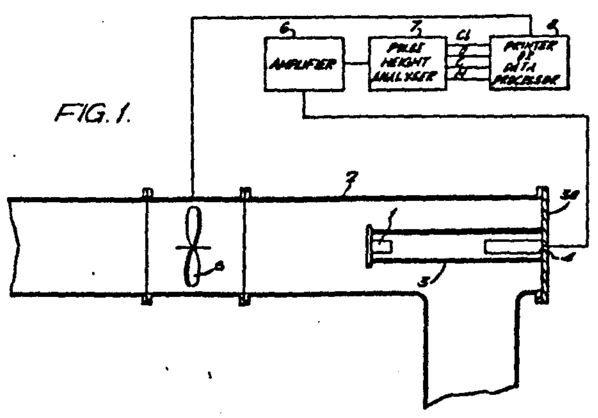


EP 0 007 759 A1

Croydon Printing Company Ltd.

TLE MODIFIED
see front page



Improvement in or relating to the measurement of the Water Content of Crude Oil

The present invention relates to the measurement of the water content of crude oil.

Crude oil extracted from wells such as those in the North Sea may contain water at concentrations which vary from a fraction of one percent to several percent. As royalties are calculated on the basis of the net amount of oil transferred to the mainland, it is desirable to be able to establish the water content of the oil to a considerable accuracy.

The methods of measuring water content which are employed at the moment are not entirely satisfactory. The settling tank method is slow and inaccurate and requires the installation of large equipment which may be impracticable. Methods based on capacitance probes, although widely used are of doubtful accuracy in the long term due to the formation of deposits on the surfaces of the electrodes. Sampling methods are slow and mechanically complex and give rise to errors if the water is not fully mixed so that unrepresentation samples are obtained.

According to the present invention there is provided a method of measuring the water content of crude oil comprising the operations of irradiating a sample of crude oil in a fixed geometry with a known dose of neutrons, measuring the amount of $\gamma$-ray activity arising from the interaction of the neutrons with either chlorine or oxygen present in the sample, deriving the specific activity of the $\gamma$-rays from the sample, and comparing the specific activity of the $\gamma$-rays from oxygen or chlorine in the sample with that arising from a known concentration of oxygen or chlorine in crude oil.

Also according to the invention there is provided an apparatus for measuring the water content of crude oil, comprising means for irradiating a sample of crude oil in a

fixed geometry with a known dose of neutrons, means for measuring the activity of Y-rays arising from the neutrons interacting with either oxygen or chlorine present in the sample, and means whereby the specific activity of the Y-rays from the oxygen or chlorine in the sample can be compared with that arising from a known concentration of oxygen or chlorine in crude oil.

The specific activities of known concentrations of oxygen or chlorine in crude oil are derived from calibration experiments.

The Y-rays arising from oxygen enable the water-content of the crude oil to be measured at all times, whereas the Y-rays arising from chlorine can only be utilised if the water is saline, but in this case, it is the preferred way of carrying out the invention.

Additionally prompt Y-rays arising from inelastic scattering of neutrons by $^{12}C$ (4.43 MeV) and from neutron capture by $^{1}H$ (2.223 MeV) occur, and, if desired these can be used to measure H:C:O ratios. Also, other elemental concentrations, such as the sulphur content, can be measured using Y-rays arising from neutron capture, and these provide valuable information about the composition of crude oil.

In one embodiment of the invention the Y-rays have an energy of 6.13 MeV and result promptly from the inelastic scattering of neutrons by the oxygen nuclei in the water. In a second embodiment of the invention, although the Y-rays again have an energy of 6.13 MeV, their emission is delayed and results from the decay of $^{16}N$ nuclei which are produced by high energy neutrons interacting with the $O^{16}$ nuclei in the water. In a third embodiment of the invention, the Y-rays arise promptly from the capture of thermal neutrons by the nuclei of $^{35}Cl$ present in the water in the crude oil. Y-rays of many energies are produced in this reaction, but the most intense lines are at 6.111, 6.620, 7.414 and 7.790 MeV.

The invention will now be described, by way of example, with reference to the accompanying drawings, in which:

Figure 1 is a block circuit diagram of an apparatus for use in carrying out the invention, and

Figures 2 to 8 illustrate various alternative configurations of neutron sources and γ-ray detectors for use in the apparatus of Figure 1.

Referring to Figure 1, an apparatus for determining the water concentration of crude oil comprises a $^{241}$Am-Be neutron source 1, supported in a pipe 2, through which crude oil is flowing, by an open structure 3 which also supports a NaI (Tl), scintillation γ-ray detector. The structure 3 is mounted on a plate 3a which is part of an elbow in the pipe 2. Also situated in the pipe 2 is a flowmeter 5. The output signal from the γ-ray detector 4 is applied to an amplifier 6 and thence to a pulse height analyser 7. The pulse height analyser 7 is arranged to produce four output signals corresponding to γ-ray counts arising from oxygen atoms, chlorine atoms, carbon and hydrogen atoms, respectively, in the irradiated sample of oil. These output signals are fed to a data processor 8, to which also is applied a signal from the flowmeter 5 representative of the volume of crude oil which has been irradiated by neutrons from the source 1. The data processor 8 is arranged either to produce a print-out of the specific γ-activity corresponding to the concentrations of one or more of the elements oxygen, chlorine, carbon and hydrogen in the sample which can be compared by an operator with previously prepared calibration charts to arrive at a measure of the concentration of water in the crude oil, and/or C:H:O ratio if required, or the comparison can be done electronically within the data processor 8 and a direct indication of the required concentrations given.

In an alternative static system, the volume of the sample of oil can be obtained either by weighing the sample, or by knowing the volume of its container. In each case an appropriate signal is fed to the data processor 8.

Three of the ways in which the apparatus described can be used are as follows:

Method 1

The specific activity of γ-rays of energy 6.13 MeV is measured to determine the amount of oxygen present in the sample of oil. The γ-rays are de-excitation γ-rays and result from inelastic neutron scattering from $^{16}O$. The relevant reaction is $^{16}O\ (n, n'\ \gamma)\ ^{16}O$.

This method has the advantage that the γ-rays are emitted within about $10^{-10}$ secs of the excitation of the relevant nuclei. Hence, they provide an instantaneous measure of the oxygen content of the oil.

Prompt γ-rays from inelastic scattering by $^{12}C$ (4.43 MeV) and from neutron capture by $^{1}H$ (2.223 MeV) are also present in the γ-ray spectrum. These can be used to measure H:C:O ratios, if desired.

Method 2

The specific activity of 6.13 MeV γ-rays with a half-life of 7.1 secs is measured, again to determine the amount of oxygen present in the sample.

The γ-rays, although having the same energy as those used for method 1, result from the activation of $^{16}O$ to produce $^{16}N$ which subsequently decays back to $^{16}O$. The relevant reaction is

$$^{16}O\ (n,p)\ ^{16}N$$

$$^{16}N \longrightarrow {}^{16}O + \beta + \gamma ; \tau_{\frac{1}{2}} = 7.1 \text{ secs}$$

This method has the advantage, when used to measure the oxygen present in oil flowing through a pipeline, that the relatively large separation between the neutron source 1 and the detector 4 facilitates the observation of the $^{16}N$ decay and significantly reduces the background of neutron interactions in the detector 4. This improves the signal/background ratio compared to that of method 1, and consequently gives a lower limit of detection. To provide neutrons of sufficiently high energy, generally a neutron tube generating neutrons having an energy of 14 MeV is required.

The arrangement used for this technique can also be used to determine the concentrations of sodium, sulphur and chlorine by measuring the decay of activity produced by similar (n, p) and (n, $\alpha$) reactions on $^{23}Na$, $^{34}S$ and $^{37}Cl$.

Method 3

This method is of use when the water present in the oil is known to be saline. It makes use of prompt $\gamma$-rays which arise from the capture of thermal neutrons by $^{35}Cl$. The relevant reaction is

$$^{35}CL\ (n,\ \gamma)\ ^{36}Cl.$$

The cross-section for thermal neutron capture by $^{35}Cl$ is two orders of magnitude greater than the cross-sections for the reactions employed in methods 1 and 2, so that a greater $\gamma$-ray intensity is produced. Moreover, the thermal neutron flux falls off less rapidly with distance from the neutron source 1 than does the fast neutron flux so that a larger volume of oil can be sampled in a given time than can be done by the other two methods. Also, because of the larger signal/background ratio when this method is used, the demand on the performance of the associated electronic equipment is less severe than in the previous two methods, particularly so far as the gain stability of the amplifier 6 is concerned.

However, an additional measurement has to be made to establish the salinity of the water.

The method does rely on the salinity of the water remaining reasonably constant, but generally this is true for a given reservoir. In practice, occasional measurements of the salinity of the water can be made to establish any trend in saline concentration. The γ-rays follow neutron capture within about $10^{-14}$ secs, so like method 1 this method is not sensitive to changes in the rate of flow of the oil.

Methods 1 and 2 of measuring the water content of crude oil require a high energy source of neutrons. Possible choices include isotopic sources such as the $^{241}$Am-Be source described with reference to Figure 1, and accelerator sources, for example based on the $^{3}$H (d,n) $^{4}$He reaction. Method 3 of measuring the water content of crude oil can also use a spontaneous fission neutron source such as that which makes use of $^{252}$Cf.

The signal/background ratio differs between various types of detector, and the choice will depend on the application. Generally, inorganic scintillation detectors such as NaI (Tl) are satisfactory, but semiconductor detectors such as intrinsic Ge detectors, or organic scintillation detectors such as those known by the code number NE-213, can be used.

Figures 2 to 8 illustrate various other source-detector geometries which can be used. The main constraint is that the separation of the source 1 from the detector 4 should be such as to optimise the signal/background ratio at the detector 4. For example, although the arrangement shown in Figure 1 can be used for all three methods of measuring the water content of oil, it is particularly suitable for methods 1 and 3.

Figure 2 shows another source-detector arrangement which is suitable for use in carrying out methods 1 or 3 of measuring the water content of crude oil. In this arrangement the

the neutron source 1 is again mounted in the pipe 2 by an open structure 3 so that it is surrounded by the oil the water content of which is to be measured, but in this arrangement the detector 4 is mounted outside the pipe in radial alignment with the neutron source 1.

Figure 3 shows another source-detector arrangement for use in carrying out methods 1 and 3 of measuring the water content of crude oil. In this arrangement both the neutron source 1 and the detector 4 are attached to the outside of the pipe 2. The neutron source 1 can, in this case, be an accelerator type source if required. It is necessary, however, to provide it with a shield 31 to absorb neutrons in directions other than directly through the oil in the pipe 2 to the detector 4.

Figure 4 illustrates another source-detector arrangement for use in carrying out methods 1 and 3 of measuring the water content of crude oil. The source 1 is positioned on the axis of the pipe 2, either by means of a structure 41 mounted on a plate 41a which forms part of an elbow in the pipe 2, such as previously illustrated, or by being placed in a tube 42 which is inserted radially through the wall of the pipe 2. In either case, four detectors 4 are disposed regularly around the pipe 2 in radial alignment with the neutron source 1.

Figure 5 illustrates a source-detector arrangement which can be used for any of methods 1, 2 and 3. The neutron source 1 and the detector 4 are inserted into tubes 51 and 52, respectively, which pass through the wall of the pipe 2. The neutron source 1 and the detector 4 are preffered to be in alignment longitudinally of the pipe 2, preferably on its axis. The separation of the detector 4 from the neutron source 1 is chosen to fulfil the signal/background criterion previously referred to; for method 2 it will be considerably greater than for methods 1 or 3.

Figure 6 illustrates a second source-detector arrangement which can be used for methods 1, 2 and 3 of measuring the water content of crude oil. It is analogous to the arrangement in Figure 5 but both the neutron source 1 and the detector 4 are attached to the wall of the pipe 2. As in the arrangement of Figure 3, it is necessary to provide the source 1 with a shield 60 to absorb unwanted neutrons.

Figure 7 shows an arrangement which is basically similar to that of Figure 1, except that the region of the pipeline 2 in which the measuring device is incorporated is expanded to form a mixing chanber 61, and that two high pressure pumps 62 are arranged to draw crude oil from that which is passing along the pipeline 1 and inject it into the mixing chamber 61 through nozzles 63 so as to ensure that the crude oil mixture passing through the pipeline 1 is well-mixed before it is analysed for its water content. Also, the neutron source and Y-ray detector assembly 4 are no longer immersed in the crude oil but are contained in a separate module 64 which is inserted in a housing 65 which projects from the end wall 66 of the mixing chamber 61. The neutron source 1 and the Y-ray detector assembly 4 are arranged in a back-scatter geometry. A baffle 67 constrains the crude oil to flow over the surface of the housing 65 before it can leave the mixing chamber 61. The mixing chamber 61 is surrounded by a biological radiation shield 68. On the outlet side of the mixing chamber 61 there is provided a sampling facility 69 by means of which quantities of the crude oil flowing through the pipeline 1 can be drawn off for calibration and other test purposes.

Figure 8 is a representation of another apparatus in which steps are taken to ensure that the crude oil flowing in the pipeline 1 are well and truly mixed before the water content is measured. Referring to Figure 8, the apparatus has inlet and outlet pipes 71 and 72 which are of the same diameter as the main pipeline 2, and which can be connected to the pipeline 2 by means of flanges 73 and 74, respectively. The inlet

pipe 71 feeds into four regularly spaced subsidiary pipes 75, the total area of which is approximately the same as that of the inlet and outlet pipes 71 and 72. The subsidiary pipes 75 recombine into the outlet pipe 72. Each of the subsidiary pipes 75 contains a helical mixing device 76 to ensure thorough mixing of the crude oil flowing in the pipeline 2. The downstream ends of the subsidiary pipes 75 and the region where they recombine into a single pipe are surrounded by a lead fast neutron reflector 77. In the space between the subsidiary pipes 75 there is situated a measuring module 78 consisting of an appropriate neutron source 79, a shadow shield 80, a sodium iodide crystal 81 and a photomultiplier tube 82 the output of which is connected to a data processing arrangement similar to that used for the Figure 1 embodiment, and which is not illustrated further. The region between the inlet and outlet pipes 71 and 72 is surrounded by a moderator and biological radiation shield 83.

Claims

1. A method of measuring the water content of crude oil comprising the operations of irradiating a sample of crude oil in a fixed geometry with a known dose of neutrons, measuring the amount of $\gamma$-ray activity arising from the interaction of the neutrons with either chlorine or oxygen present in the sample, deriving the specific activity of the $\gamma$-rays from the sample, and comparing the specific activity of the $\gamma$-rays from oxygen or chlorine in the sample with that arising from a known concentration of oxygen or chlorine in crude oil.

2. A method according to claim 1 wherein the $\gamma$-rays which are measured are those which arise promptly from the capture of thermal neutrons by nuclei of $^{35}Cl$ present in any water present in the crude oil, and there is included the further operation of determining the salivity of the water.

3. A method according to claim 1 wherein the $\gamma$-rays which are measured result promptly from the inelastic scattering of neutrons by the oxygen nuclei in any water which is present in the crude oil.

4. A method according to claim 1 in which the $\gamma$-rays which are measured are delayed and result from the radioactive decay of nuclei of $^{16}N$ which are produced by high energy neutrons interacting with the nuclei of $^{16}O$ from any water which is present in the crude oil.

5. A method according to claim 3 or claim 4 wherein there is included the operations of measuring the specific activities of prompt $\gamma$-rays resulting from inelastic scattering of neutrons by $^{12}C$ nuclei present in the crude oil and from neutron capture by $^{1}H$ present in the crude oil, thereby to determine the ratios of hydrogen to carbon to oxygen in the crude oil.

6. An apparatus for measuring the water content of crude oil, comprising means for irradiating a sample of crude oil in a fixed geometry with a known dose of neutrons, means for measuring the activity of γ-rays arising from the neutrons interacting with either oxygen or chlorine present in the sample, and means whereby the specific activity of the γ-rays from the oxygen or chlorine in the sample can be compared with that arising from a known concentration of oxygen or chlorine in crude oil.

7. An apparatus according to claim 6 wherein there is included means for agitating the crude oil prior to irradiation so as to ensure that any water present in the crude oil is distributed uniformly throughout the crude oil.

8. Apparatus according to claim 6 or claim 7 wherein the means for measuring the specific γ-ray activity comprises means for producing light pulses in response to γ-rays arising from nuclear interaction with the oil, means for producing electrical pulses related to said light pulses, a pulse height analyser arranged to produce output signals corresponding to γ-ray counts arising from oxygen or chlorine nuclei within the crude oil, means for producing a signal related to the quantity of crude oil which has been irradiated with neutrons, a data processor to which the signals corresponding to the said γ-ray counts and the quantity of crude oil which has been irradiated are applied and which is arranged to derive the specific activity of the γ-rays arising from oxygen or chlorine nuclei in the crude oil.

9. Apparatus according to claim 8 wherein the data processor is arranged to compare the derived specific activities of oxygen or chlorine with the specific activities of known concentrations of pure or saline water in crude oil and provide a direct indication of the concentration of water in the crude oil.

10. Apparatus according to claim 8 or claim 9 wherein the pulse height analyser is arranged to produce further output signals related to Y-ray counts arising from nuclei of carbon and hydrogen in the crude oil, and the data processor is arranged to derive the ratios of carbon to hydrogen to oxygen in the crude oil.

11. Apparatus according to claim 7 wherein there is provided a mixing chamber having a housing in an end wall in which there may be inserted a measuring module consisting of a neutron source and a Y-ray detector assembly arranged in a back-scatter geometry, a baffle system arranged to cause crude oil passing through the mixing chamber to pass over the surface of the housing thereby to enable the crude oil to be irradiated with neutrons, means for connecting the mixing chamber into a pipeline through which the crude oil the water content of which is to be measured can be caused to flow, and a plurality of pumps arranged to draw crude oil from the pipeline and inject it into the mixing chamber in a turbulent fashion thereby to ensure homogeneity in the crude oil passing through the mixing chamber.

12. Apparatus according to claim 7 wherein there is included a mixing device consisting of an inlet pipe and an outlet pipe which are adapted to be connected into a pipeline through which crude oil the water content of which is to be measured can be caused to flow, a plurality of subsidiary pipes of equal cross-section which are fed by the inlet pipe and which in turn feed into the outlet pipe, each of the subsidiary pipes containing a system of baffles arranged to cause turbulence in crude oil flowing through the subsidiary pipes, the subsidiary pipes being symmetrically disposed about a measuring module consisting of a neutron source and a Y-ray detector assembly arranged in a back-scatter geometry with the neutron source positioned in the region of the confluence of the subsidiary pipes and the outlet pipes.

13. A method of measuring the water content of crude oil substantially as hereinbefore described with reference to the accompanying drawings.

14. An apparatus for measuring the water content of crude oil substantially as hereinbefore described with reference to any of Figures 1 to 8 of the accompanying drawings.

On behalf of the Applicants

P.A. Wood
European Patent Attorney

11563 WdH

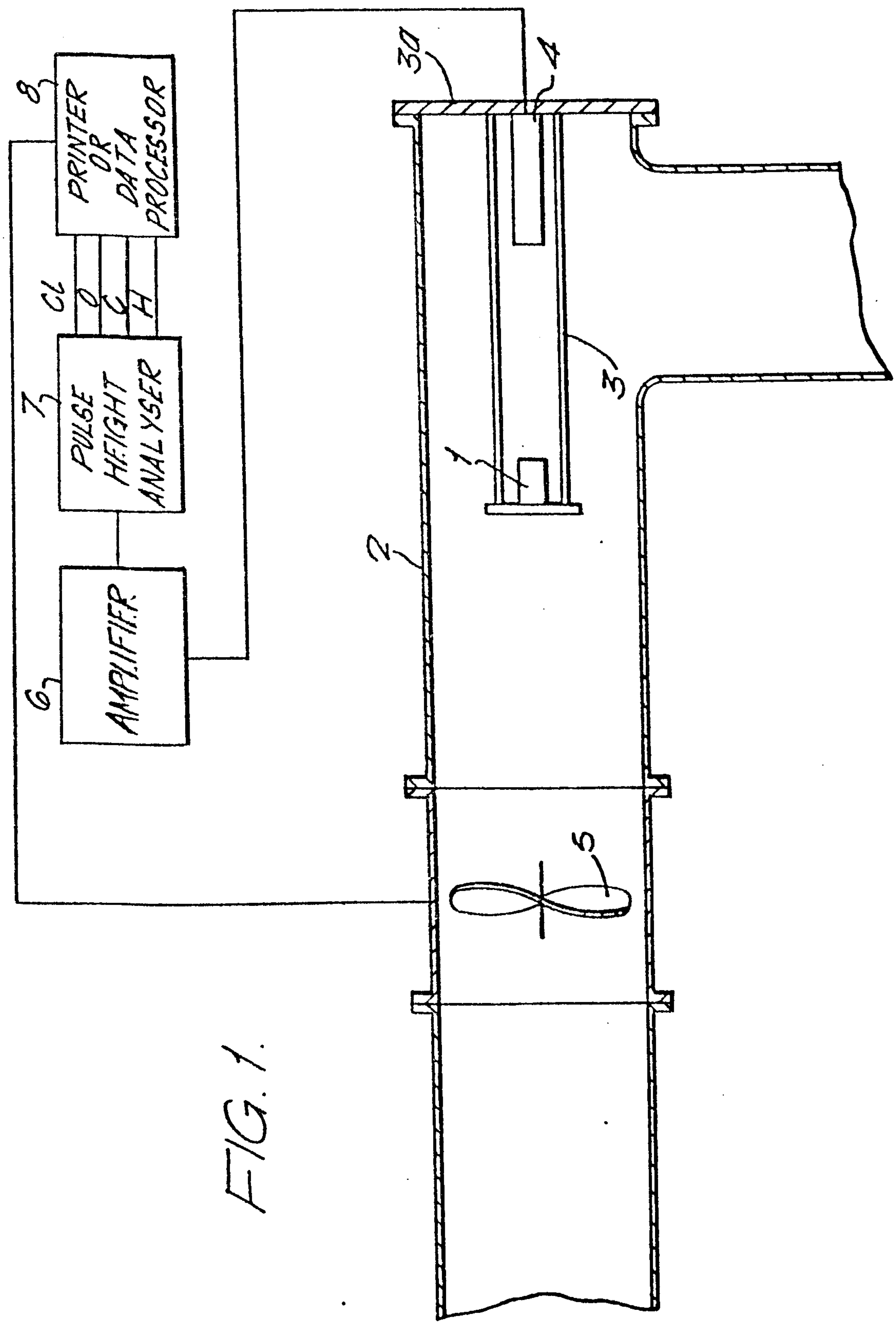
8
PRINTER OR DATA PROCESSOR
Cl
O
C
H
7
PULSE HEIGHT ANALYSER
6
AMPLIFIER
3a
4
3
1
2
5

FIG. 1.

FIG. 2.

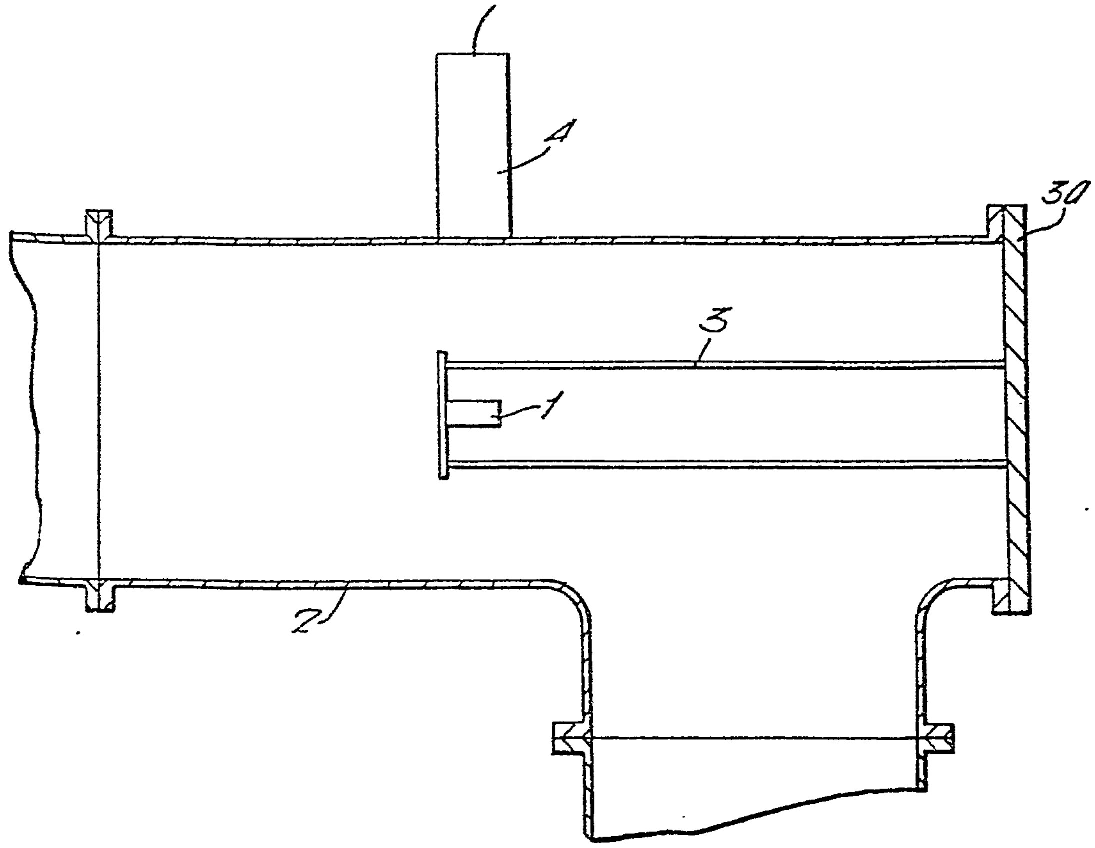


FIG. 3.

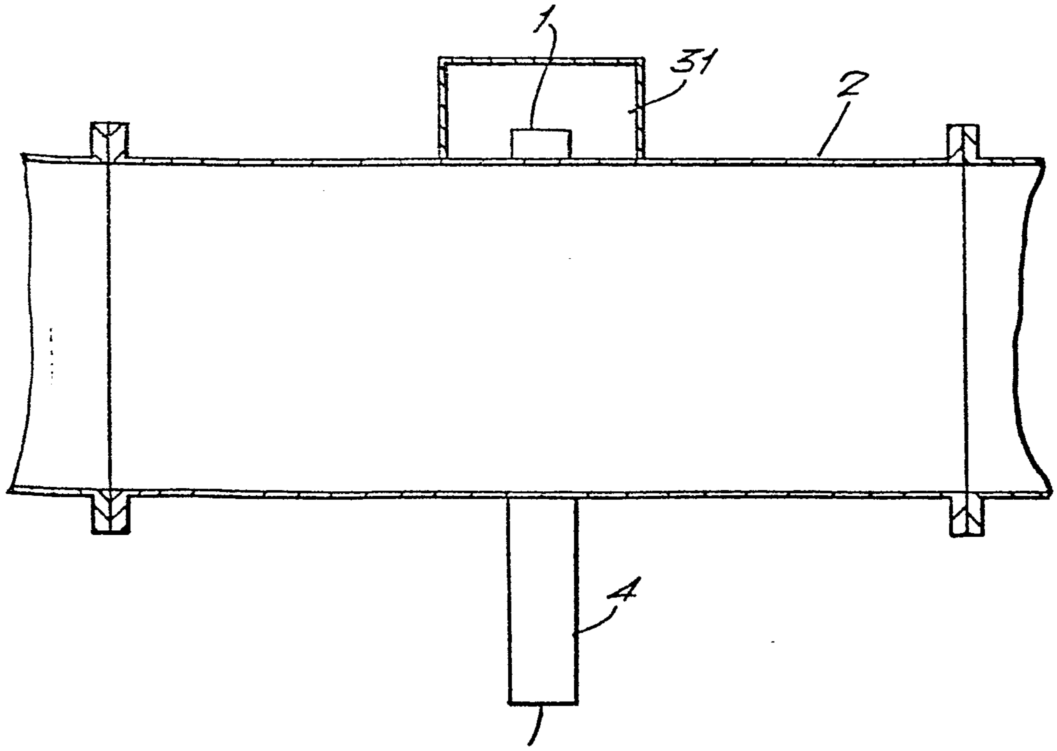

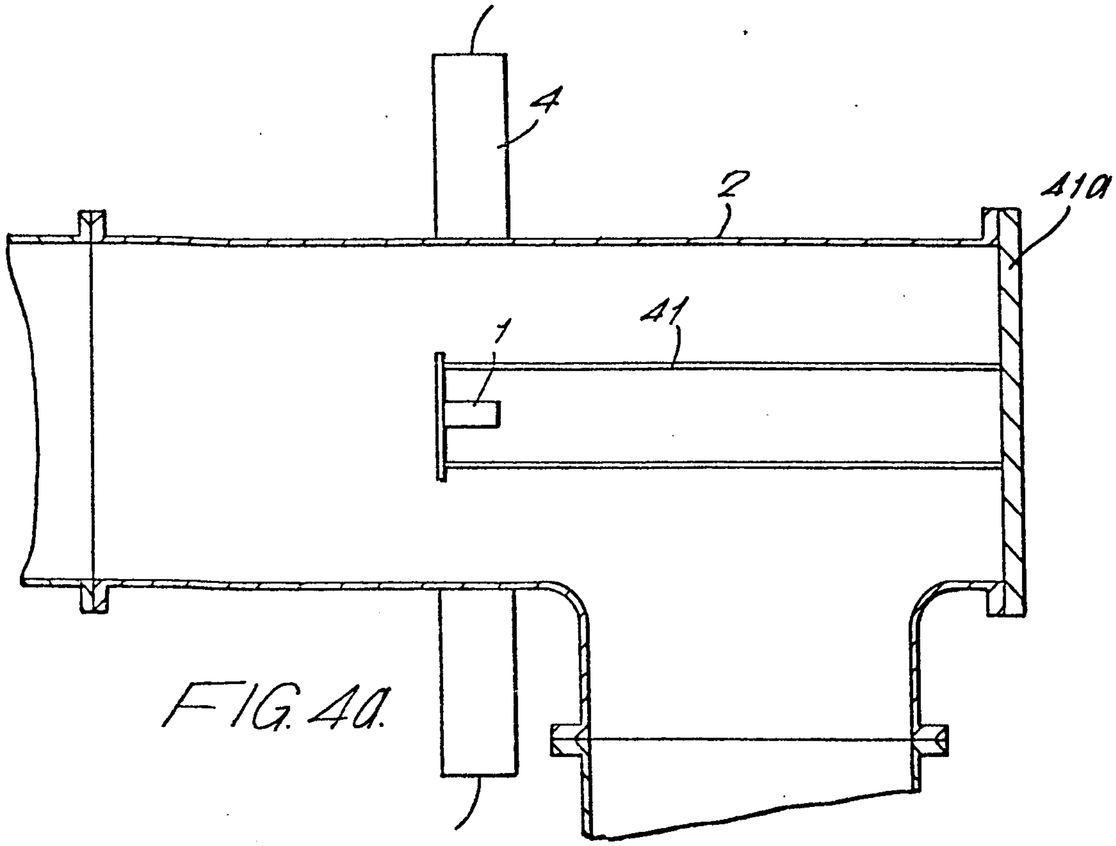


FIG. 4a.

FIG. 4b.

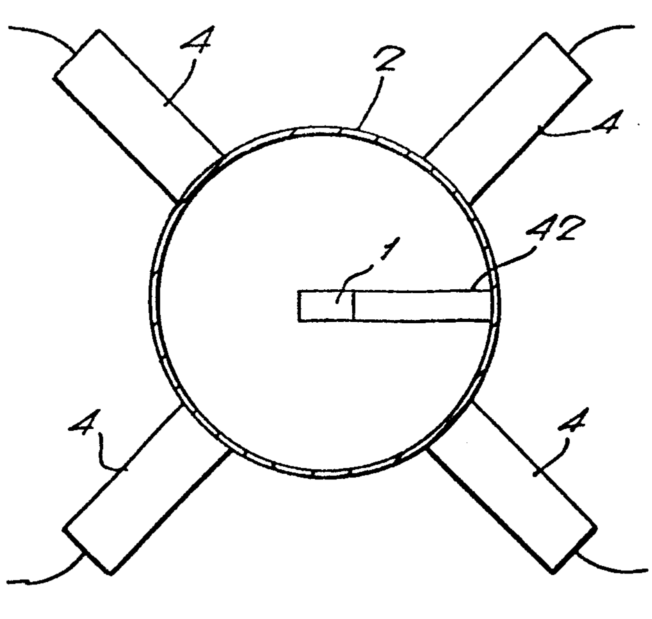

4/6

FIG. 5.

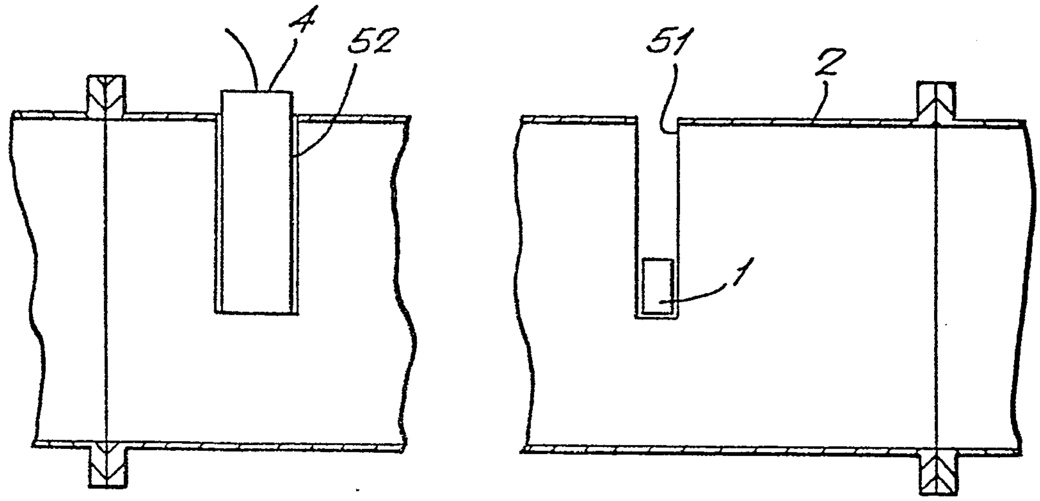


FIG. 6.

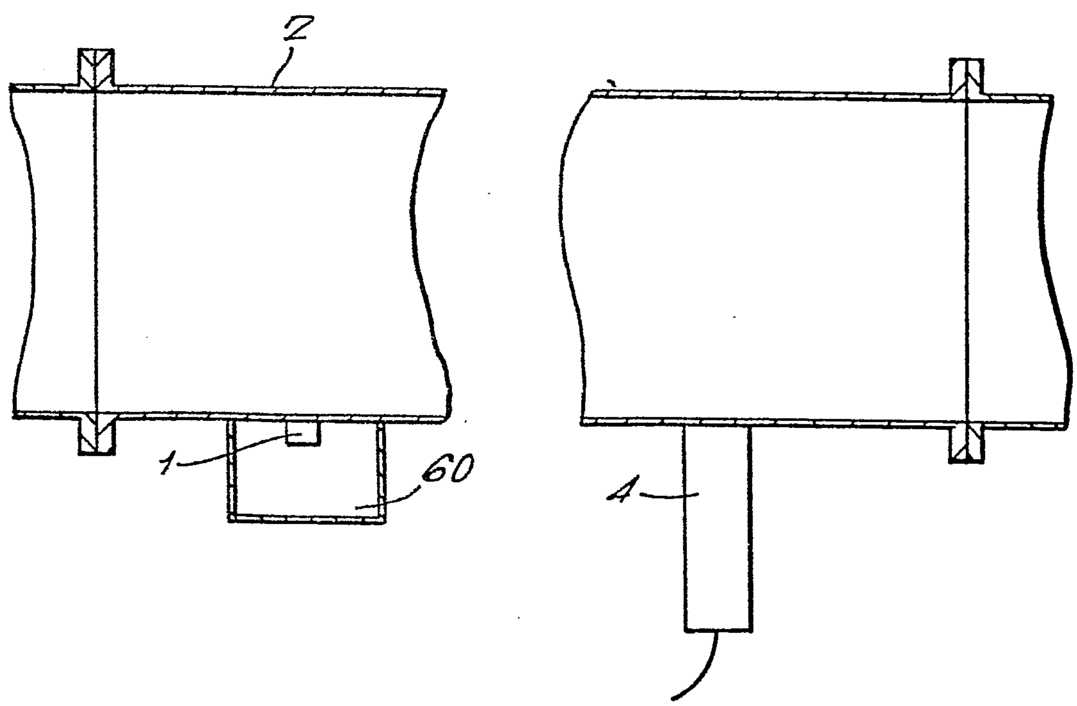

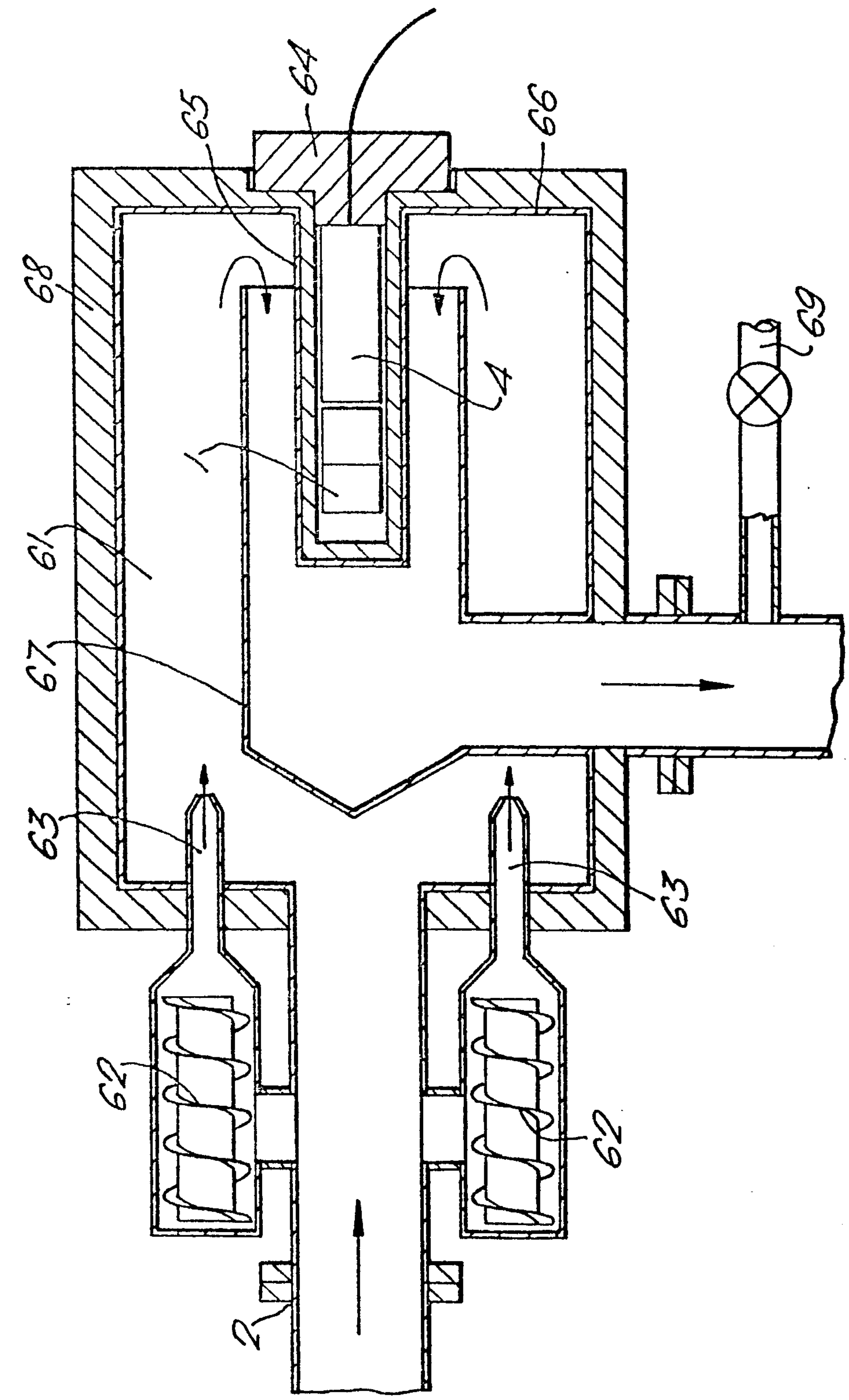


FIG. 7.

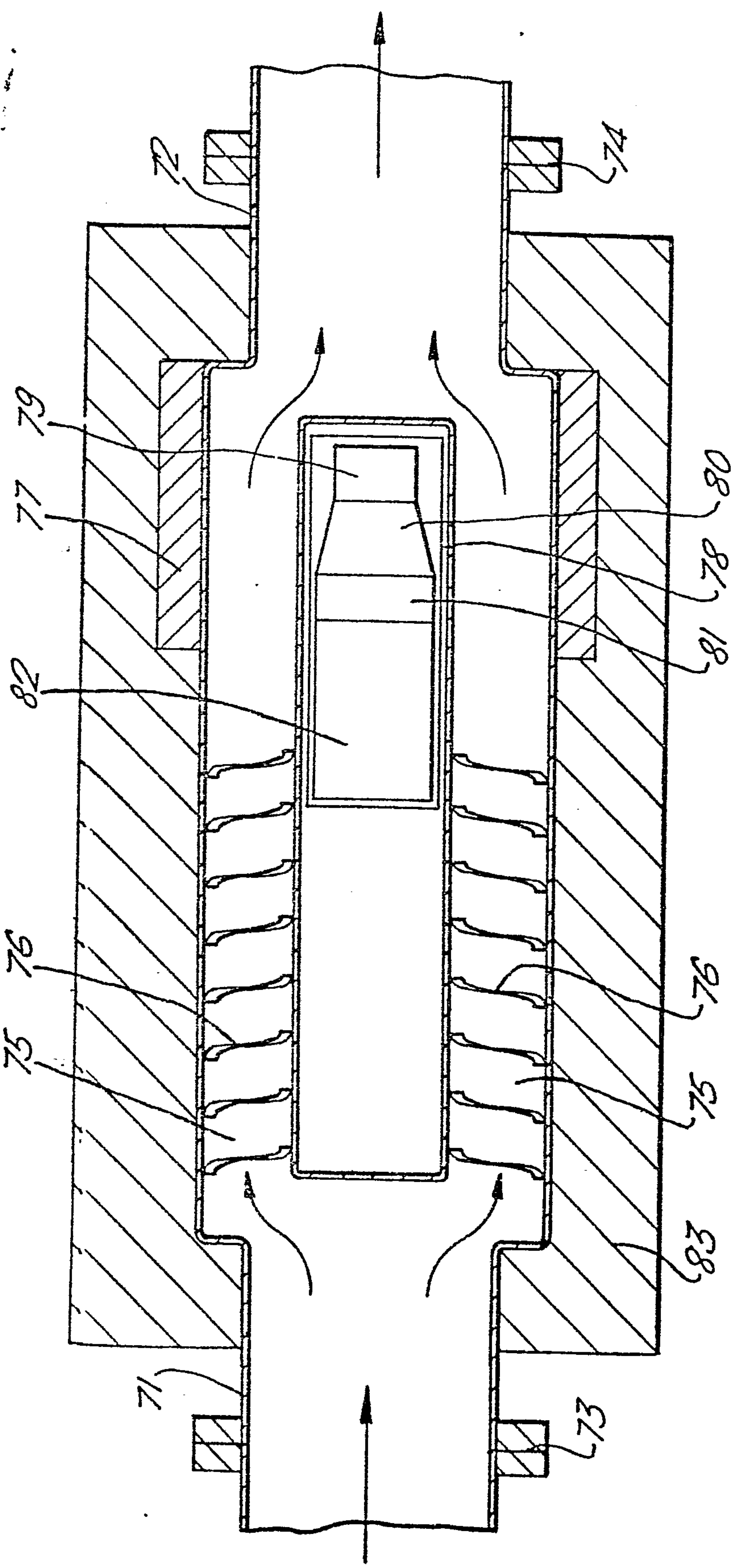


FIG. 8.

European Patent Office

# EUROPEAN SEARCH REPORT

Application number

EP 79 301 400.2

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.) |
|---|---|---|---|
| X | DE - A1 - 2 754 143 (TEXACO DEVELOPMENT)<br>* whole document *<br>& FR - A1 - 2 373 055<br>-- | 1,2, 6-9 | G 01 N 23/222<br>G 01 N 33/28 |
| | DE - A1 - 2 624 937 (GESELLSCHAFT FÜR KERNFORSCHUNG)<br>* claims 1 to 4; fig. *<br>-- | 6,7, 11,12 | |
| A | DE - B - 1 598 584 (HILGER & WATTS)<br>* whole document *<br>-- | | TECHNICAL FIELDS SEARCHED (Int.Cl.) |
| A | US - A - 3 823 316 (UNITED KINGDOM ATOMIC ENERGY AUTHORITY)<br>* whole document *<br>-- | | G 01 N 23/22<br>G 01 N 33/28 |
| | CHEMISCHE TECHNIK, Vol. 28, Nr. 4, 1976, Weinheim<br>G. BRUNNER "Aktivierungsanalyse" pages 218 to 223<br>* whole document *<br>-- | | |
| A | ANALYTICAL CHEMISTRY, Vol. 50, Nr. 7, June 1978<br>Columbus, Ohio, USA,<br>T. GERARD et al. " Determination of halogens by activation analysis with a californium-252 neutron multiplier"<br>pages 906 to 910<br>* whole document *<br>-- ./.. | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |
| X | The present search report has been drawn up for all claims | | |

| Place of search | Date of completion of the search | Examiner |
|---|---|---|
| Berlin | 23-10-1979 | SCHWARTZ |

0007759

European Patent Office

**EUROPEAN SEARCH REPORT**

Application number
EP 79 301 400.2
- page 2 -

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | ANALYTICAL CHEMISTRY, Vol. 46, Nr. 9 1974<br>Columbus, Ohio, USA,<br>A.R. POURAGHABAGHER et al. "Neutron capture gamma ray spectrometry for determination of sulfur in oil"<br>pages 1223 to 1226<br>* whole document *<br>---- | | |
| | | | TECHNICAL FIELDS SEARCHED (Int. Cl.3) |